Europäisches Patentamt
European Patent Office
Office européen des brevets

(11) Publication number: **0 070 824**
**B2**

(12) **NEW EUROPEAN PATENT SPECIFICATION**

(45) Date of publication of the new patent specification: **03.10.90**

(51) Int. Cl.[5]: **B 01 J 29/04,** B 01 J 29/16, B 01 J 37/10, B 01 J 37/04, C 10 G 11/05, C 10 G 45/12, C 10 G 47/16

(21) Application number: **81901603.1**

(22) Date of filing: **09.02.81**

(86) International application number: **PCT/US81/00174**

(87) International publication number: **WO 82/02677 19.08.82 Gazette 82/20**

(54) **HYDROCARBON CONVERSION CATALYST.**

(43) Date of publication of application: **09.02.83 Bulletin 83/06**

(45) Publication of the grant of the patent: **05.02.86 Bulletin 86/06**

(45) Mention of the opposition decision: **03.10.90 Bulletin 90/40**

(84) Designated Contracting States: **AT DE FR GB NL**

(56) References cited:
**EP-A-0 028 938**
**DE-A-2 036 821**
**US-A-3 251 902**
**US-A-3 277 018**
**US-A-3 542 670**
**US-A-3 617 483**
**US-A-3 729 521**
**US-A-3 781 199**
**US-A-3 783 124**
**US-A-3 835 027**
**US-A-3 836 454**
**US-A-3 867 277**
**US-A-3 926 780**
**US-A-3 974 099**
**US-A-3 997 618**
**US-A-4 036 739**
**US-A-4 062 809**
**US-A-4 097 365**
**US-A-4 115 248**
**US-A-4 120 825**
**US-A-4 182 693**
**US-A-4 238 361**
**US-A-4 242 237**
**US-A-4 246 138**
**US-A-4 515 121**

**The file contains technical information submitted after the application was filed and not included in this specification**

(73) Proprietor: **UNION OIL COMPANY OF CALIFORNIA**
**376 So. Valencia Avenue P.O. Box 76**
**Brea, CA 92621 (US)**

(72) Inventor: **WARD, John W.**
**19002 Gordon Lane**
**Yorba Linda, CA 92686 (US)**

(74) Representative: **Madgwick, Paul Roland et al**
**Ladas & Parry Isartorplatz 5**
**D-8000 München 2 (DE)**

(56) References cited:
**M. Reno et al., UOP Hydrocracking Catalyst Technology, UOP-Publication, Aug. 1988**

**EP 0 070 824 B2**

Courier Press, Leamington Spa, England.

## Description

Technical field

This invention relates to hydrocarbon conversion processes and catalysts for use therein. More particularly, it relates to a composition of matter for catalyzing acid hydrocarbon conversion reactions and to a catalyst composition which comprises in intimate heterogeneous mixture a crystalline aluminosilicate zeolite having activity for catalytically cracking hydrocarbons and a silica-alumina in an alumina matrix.

Background art

Petroleum refiners often produce desirable products as turbine fuel, diesel fuel, and other middle distillate products by hydrocracking a heavy gas oil, i.e., a hydrocarbon fraction having a boiling point range between about 700°F and 1050°F (371° and 566°C). Hydrocracking is accomplished by contacting the heavy gas oil at an elevated temperature and pressure in the presence of hydrogen and a suitable hydrocracking catalyst so as to yield a middle distillate fraction boiling in the 300° to 700°F (149° to 371°C) range and containing the desired turbine and diesel fuels.

The three main catalytic properties by which the performance of a hydrocracking catalyst for producing middle distillate products is evaluated are activity, selectivity and stability. Activity may be determined by comparing the temperature at which various catalysts must be utilized under otherwise constant hydrocracking conditions with the same feedstock so as to produce a given percentage (usually 60%) of the products boiling below 700°F (371°C). The lower the activity temperature for a given catalyst, the more active such a catalyst is in relation to a catalyst of higher activity temperature. Selectivity of hydrocracking catalysts may be determined during the foregoing described activity test and is measured as that percentage fraction of the 700°F minus (371°C) product boiling in the range of middle distillate or midbarrel products, i.e., 300° to 700°F (149° to 371°C). Stability is a measure of how well a catalyst maintains its activity over an extended time period when treating a given hydrocarbon feedstock under the conditions of the activity test. Stability is generally measured in terms of the change in temperature required per day to maintain a 60% or other given conversion.

As could be expected, the aim of the art is to provide a catalyst having at once the highest possible activity, selectivity, and stability. Catalysts usually utilized for hydrocracking comprise a Group VIII metal component, most often cobalt or nickel sulfides, in combination with a Group VIB metal component, most often molybdenum or tungsten sulfides, supported on a refractory oxide. For given proportions of Group VIII and Group VIB metal components, the activity, selectivity, and stability of a catalyst change dramatically with different supports. Support materials comprising crystalline aluminosilicate zeolites, such as Zeolite Y in the hydrogen form, generally provide high activity but low selectivity, whereas support materials consisting essentially of refractory oxides, such as alumina, magnesia, and silica-alumina, generally have relatively poor activity but high selectivity.

The object of the present invention, therefore, is to provide a hydrocracking catalyst having superior overall catalytic properties for hydrocracking hydrocarbons. More specifically, it is an object of the invention to provide a catalyst having superior overall activity, selectivity, and stability for hydrocracking in comparison to prior art catalysts. It is a further object to provide a hydrocracking process for converting gas oils and the like to middle distillate products. These and other objects and advantages will become more apparent in light of the following description of the invention.

Disclosure of invention

The present invention is an improvement of the catalyst described in US—A—4,097,365, herein incorporated by reference. The catalyst described in this patent is midbarrel hydrocracking catalyst comprising hydrogenation components on a refractory oxide support comprising silica-alumina dispersed in a matrix of alumina. The present invention improves this catalyst by including in the support a crystalline aluminosilicate zeolite having cracking activity, such as hydrogen Y zeolite or a rare earth-exchanged Y zeolite. In addition to having excellent activity for hydrodenitrogenation and hydrodesulfurization, the catalyst of the invention has been found to have superior overall properties of activity, selectivity and stability for hydrocracking in comparision to the catalyst described in U.S. Patent No. 4,097,365. In the usual instance, the catalyst of the invention is more active, more stable, and more selective than comparison catalysts having supports consisting essentially of either a dispersion of silica-alumina in an alumina matrix or a zeolite plus a refractory oxide other than a dispersion of silica-alumina in an alumina matrix.

US—A—3,542,670 discloses a hydrocarbon cracking catalyst, as opposed to a hydrocracking catalyst, for use in cracking higher boiling hydrocarbons to obtain gasoline. The catalyst system includes a mixture of a zeolite having activity for catalytically cracking hydrocarbons and a matrix comprising a silica-alumina hydrogel with an amorphous hydrous alumina or an alumina monohydrate of a specified crystal size. Sodium ions may be removed from the zeolite prior to its mixture with the matrix by ion exchanging the zeolite with ammonium ions, or polyvalent metals such as Group IIA metals or rare earth metals. The patent, however, nowhere suggests the possibilities or advantages of using a zeolite that is a hydrophobic modified hydrogen crystalline aluminosilicate Y zeolite having a water absorption capacity of 4.6 mm water vapor partial pressure and 25°C less than 8 percent by weight of the zeolite or that the mixture of zeolite and matrix may support at least one hydrogenation component selected from the group consisting of Group VIII and Group VIB metals, their oxides and sulfides.

In one embodiment, the present invention provides hydrocarbon cracking catalysts in which a base supports active hydrogenation metal components said base comprising in intimate heterogenous mixture a crystalline aluminosilicate zeolite having activity for catalytically cracking hydrocarbons and silica-alumina in an alumina matrix, characterized by (1) said zeolite comprising a modified hydrogen crystalline aluminosilicate Y zeolite having a water absorption capacity at 4.6 mm water vapor partial pressure and 25°C less than 8 percent by weight of the zeolite and (2) said silica-alumina in an alumina matrix comprising a dispersion of silica-alumina in a gamma alumina matrix.

Modes, including best mode, for carrying out the invention

The catalyst of the invention is an intimate composite of one of more hydrogenation components, a crystalline aluminosilicate zeolite having catalytic activity for cracking hydrocarbons, and a dispersion of silica-alumina in a matrix consisting essentially of gamma alumina. The hydrogenation components useful in the invention are the metals, oxides, and sulfides of the Group VIII elements, and the Group VIB elements. The most suitable hydrogenation components are selected from the group consisting of the metals, oxides, and sulfides of platinum, palladium, cobalt, nickel, tungsten, and molybdenum. The preferred catalyst contains at least one Group VIII metal component, and at least one group VIB metal component, with the most preferred combination being a nickel and/or cobalt component with a molybdenum and/or tungsten component.

The hydrogenation component or components are intimately composited on a base or support comprising a mixture of one or more crystalline aluminosilicate zeolites having cracking activity and a heterogeneous dispersion of finely divided silica-alumina in a matrix of gamma alumina. The suitable zeolites for use herein include crystalline aluminosilicate molecular sieves having catalytic activity for cracking hydrocarbons. Many naturally occurring and synthetic crystalline aluminosilicate zeolites known in the art are useful in the invention, including, for example, faujasite, mordenite, erionite, Zeolite Y, Zeolite X, Zeolite L, Zeolite Omega, Zeolite ZSM—4, and their modifications. These and other such zeolitic molecular sieves are known to have activity for cracking hydrocarbons when a substantial proportion of the ion exchange sites are occupied with hydrogen ions or multivalent metal-containing cations, particularly rare earth cations. Normally, crystalline aluminosilicate zeolites are obtained in the alkali metal form and as such are largely inactive for catalytically cracking hydrocarbons. To produce a zeolite having cracking activity, the alkali metals are usually replaced with multivalent metal-containing cations, hydrogen ions, or hydrogen ion precursors (e.g. ammonium ion). This replacement of cations is generally accomplished by ion exchange, a method well-known in the art wherein the zeolite in the sodium or other alkali metal form is contacted with an aqueous solution containing hydrogen ions, ammonium ions, rare earth ions, or other suitable cations. Replacing even a portion of the sodium ions produces a zeolite having some cracking activity, but reducing the alkali metal content to less than 5 wt.%, preferably to less than 1 wt.%, and most preferably to less than about 0.5 wt.% (calculated as the alkali metal oxides), results in a material having substantial cracking activity, with the activity varying according to the zeolite and the amount of alkali metals removed.

In addition to the zeolites referred to above, many other crystalline aluminosilicate zeolites in their non-alkali metal forms may be utilized in the catalyst support of the invention. Preferred zeolites contain at least 50% of their pore volume in pores of diameter greater than 8 Angstroms, with Zeolite Y (and its modifications) in the hydrogen form or in other forms imparting cracking activity to the zeolite being preferred zeolites for use in the invention. Also preferred are zeolites that have been ion-exchanged with ammonium ions and then steam stabilized in accordance with the teachings of US—A—3,929,672, herein incorporated by reference. The most highly preferred zeolite is a material known as LZ-10, a zeolitic molecular sieve available from Union Carbide, Linde Division. Although LZ-10 is a proprietary material, it is known that LZ-10 is a modified Y zeolite having a silica to alumina ratio between about 3.5 and 6.0, a surface area between about 500 and 700 $m^2$/gm, a unit cell size between about 24.25 and 24.35 Angstroms, water absorption capacity less than about 8% by weight of the zeolite (at 4.6 mm partial pressure of water vapor and 25°C), and an ion-exchange capacity less than 20% of that of a sodium Y zeolite of comparable silica to alumina ratio. When used as a hydrocracking catalyst, LZ-10 is highly active and selective for midbarrel hydrocracking, especially when composited with alumina and suitable hydrogenation components.

The support material utilized in the invention usually comprises between 2 and about 80% by weight, preferably between about 10 and about 70% by weight, of a crystalline aluminosilicate zeolite such as LZ-10. The support also comprises a substantial proportion of a heterogeneous dispersion of finely divided silica-alumina in a gamma alumina matrix. Usually, the dispersion comprises at least 15% by weight of the support, with the preferred and most preferred proportions being in the respective ranges of 30 to 98% and 30 to 90% by weight of the support.

One convenient method of preparing the catalyst support herein is to comull an alumina hydrogel with a silica-alumina cogel in hydrous or dry form. The cogel is preferably homogeneous and may be prepared in a manner such as that described in US—A—3,210,294. Alternatively, the alumina hydrogel may be comulled with a "graft copolymer" of silica and alumina that has been prepared, for example, by first impregnating a silica hydrogel with an alumina salt and then precipitating alumina gel in the pores of the silica hydrogel by contact with ammonium hydroxide. In the usual case, the cogel or copolymer (either of

which usually comprises silica in a proportion by dry weight of 20 to 96%, preferably 50 to 90%) is mulled with the alumina hydrogel such that the cogel or copolymer comprises 5 to 75% by weight, preferably 20 to 65% by weight, of the mixture. The overall silica content of the resulting dispersion on a dry basis is usually between 1 and 75 wt.%, preferably between 5 to 45 wt.%.

The mulled mixture of alumina gel with either a silica-alumina cogel or a silica and alumina "graft copolymer" may be utilized in the gel form or may be dried and/or calcined prior to combination with the zeolite. In the preferred method of preparation, the cogel or copolymer is spray dried and then crushed to a powdered form, following which the powder is mulled with a zeolite powder containing hydrogen ions, hydrogen ion precursors, or multivalent metal-containing cations, the amount of cogel or copolymer mulled with said zeolite being such that the support will ultimately contain zeolite and dispersion in the proportions set forth hereinbefore. If desired, a binder may also be incorporated into the mulling mixture, as also may one or more active metal hydrogenation components in forms such as ammonium heptamolybdate, nickel nitrate or chloride, ammonium metatungstate, cobalt nitrate or chloride, etc. After mulling, the mixture is extruded through a die having suitable openings therein, such as circular openings of diameters between about 1/32 and 1/8 inch (0.08 to 0.32 cm). Preferably, however, the die has openings therein in the shape of three-leaf clovers so as to produce an extrudate material similar to that shown in Figures 8 and 8A of US—A—4,028,227. The extruded material is cut into lengths of about 1/32 to 3/4 inch (0.08 to 1.91 cm), preferably 1/4 to 1/2 inch (0.64 to 1.27 cm), dried, and calcined at an elevated temperature.

If desired, hydrogenation components may be composited with the support by impregnation; that is, rather than comulling the hydrogenation components with the support materials, the zeolite and dispersion are mulled, extruded, cut into appropriate lengths, and calcined. The resulting particles are then contacted with one or more solutions containing the desired hydrogenation components in dissolved form, and the composite particles thus prepared are dried and calcined to produce finished catalyst particles.

Usually, the finished catalyst contains at least about 0.5 wt.% of hydrogenation components, calculated as the metals. In the usual instance, wherein a Group VIII metal and a Group VIB metal component are utilized in combination, the finished catalyst contains between about 5% and 35%, preferably between about 10 and 30% by weight, calculated as the respective trioxides, of the Group VIB metal components and between about 2% and 15%, preferably between 3 and 10% by weight, calculated as the respective monoxides, of the Group VIII metal components.

If desired, a phosphorus component may also be incorporated in the catalyst by either comulling the support materials with phosphoric acid or including phosphoric acid in the impregnating solution. Usual and preferred proportions of phosphorus in the catalyst fall in the ranges of 1 to 10 wt.% and 3 to 8 wt.%, calculated as $P_2O_5$.

The hydrogenation components, which will largely be present in their oxide forms after calcination in air, may be converted to their sulfide forms, if desired, by contact at elevated temperatures with a reducing atmosphere comprising hydrogen sulfide. More conveniently, the catalyst is sulfided in situ, i.e., by contact with a sulfur-containing feedstock to be catalytically converted to more valuable hydrocarbons in such processes as hydrocracking, hydrotreating, etc.

The foregoing described catalysts are useful with respect to hydrocracking to convert a hydrocarbon feedstock to a more valuable product of lower average boiling point and lower average molecular weight. The feedstocks that may be treated herein by hydrogenation include all mineral oils and synthetic oils (e.g., shale oil, tar sand products, etc.) and fractions thereof. Typical feedstocks include straight run gas oils, vacuum gas oils, deasphalted vacuum and atmospheric residua, coker distillates, and catcracker distillates. Hydrocracking feedstocks include gas oils and other hydrocarbon fractions having at least 50% by weight of their components boiling above 700°F (371°C). Suitable and preferred conditions for hydrocracking gas oil feedstocks, are:

TABLE I

| | Suitable | Preferred |
|---|---|---|
| Temperature, °F (°C) | 500—850 (260—454) | 600—800 (316—427) |
| Pressure, psig | 750—3500 | 1000—3000 |
| atm | 51—234 | 68—201 |
| LHSV | 0.3—5.0 | 0.5—3.0 |
| $H_2$/Oil*, MSCF/bbl | 1—10 | 2—8 |
| cc/ml | 178—1781 | 356—1425 |

*as measured at 60°F (15.6°C)

As will be shown by the following Examples, which are provided for illustrative purposes and are not to be construed as limiting the scope of the invention as defined by the claims, the present catalysts have

been found to possess superior overall catalytic properties when conversion of gas oils to midbarrel products by hydrocracking is desired. In many instances, the catalysts of the invention have been found to be superior in each of the three main performance categories of activity, selectivity, and stability.

Example I

An experiment was performed to compare the activity, selectivity, and stability of catalysts of the invention containing LZ-10 and a dispersion of silica-alumina in a gamma alumina matrix versus catalysts having supports consisting essentially of LZ-10 and gamma alumina. Following are the preparation procedures used for Catalyst Nos. 1 through 4, with Catalyst Nos. 2 and 3 being representative of the invention and Catalyst Nos. 1 and 4 being the comparison catalysts.

Catalyst No. 1

A mixture of 10% by weight powdered LZ-10 that had been ion-exchanged with ammonium nitrate to reduce the sodium content to about 0.1% by weight sodium (as $Na_2O$) and 90% by weight gamma alumina was extruded through a die having opening therein in a three-leaf clover shape, each leaf being defined by about a 270° arc of a circle having a diameter between about 0.02 and 0.04 inches (0.05 to 0.10 cm). The extruded material was cut into $\frac{1}{4}$—$\frac{1}{2}$ inch (0.64 to 1.27 cm) lengths and calcined at 900°F (482°C) in air to convert the LZ-10 material to the hydrogen form. The calcined particles (300 gm) were then impregnated with 330 ml of an aqueous solution containing 67 gm of nickel nitrate ($Ni(NO_3)_2 \cdot 6H_2O$) and 108 gm of ammonium metatungstate (91% $WO_3$ by weight). After removing excess liquid, the catalyst was dried at 230°F (110°C) and calcined at 900°F (482°C) in flowing air. The final catalyst contained 4.4 wt.% nickel components (calculated as NiO) and 25.0 wt.% tungsten components (calculated as $WO_3$).

Catalyst No. 2

The procedure described for Catalyst No. 1 was repeated except that in place of alumina a dispersion of spray dried, powdered silica-alumina in alumina prepared in a manner similar to that of Example 3 of U.S. Patent No. 4,097,365 was used. The dispersion was prepared by mixing 44 parts by dry weight of a 75/25 silica-alumina graft copolymer and 56 parts by weight of hydrous alumina gel. In the final catalyst, the support consisted essentially of 10% LZ-10 in the hydrogen form and 90% dispersion of silica alumina in an alumina matrix, the dispersion consisting overall of 33% by weight silica and 67% by weight alumina. The resulting catalyst contained 4.1% by weight nickel components (as NiO) and 24.2% by weight tungsten components (as $WO_3$).

Catalyst No. 3

This catalyst was prepared in the same manner as Catalyst No. 2 except that the proportions of LZ-10 and dispersion admixed to prepare the support were adjusted so that in the final catalyst the proportion of LZ-10 in the support was 5% by weight and that of the dispersion, 95% by weight. The final catalyst contained 4.1% by weight nickel components (as NiO) and 23.6% by weight tungsten components (as $WO_3$) on a support of 5% LZ-10 and 95% dispersion.

Catalyst No. 4

This catalyst was prepared in the same manner as Catalyst No. 1 except that the proportions of LZ-10 and alumina admixed during preparation were such that in the final catalyst the proportion of LZ-10 in the support was 20% by weight and that of the gamma alumina, 80% by weight. The final catalyst contained 4.1% by weight nickel components (as NiO) and 24.4% by weight tungsten components (as $WO_3$) on a support of 20% LZ-10 and 80% gamma alumina.

Each of the foregoing catalysts was then activity tested according to the following method. A preheated light Arabian vacuum gas oil having the chemical and physical properties shown in Table II was passed on a once-through basis through an isothermal reactor containing 140 ml of catalyst particles uniformly mixed with 160 ml of 10 to 20 mesh quartz. Operating conditions were as follows: 1.0 LHSV, 2000 psig (135 atm), a once-through hydrogen flow of 10,000 scf/bbl (1781 cc/ml), and a run length of approximately 10 days. The temperature of the reactor was adjusted to provide a 60 volume percent conversion to products boiling at 700°F (371°C) or less. The results of the activity testing are reported in Table III.

TABLE II

Properties of light Arabian vacuum gas oil

| | |
|---|---|
| Gravity, °API 22.3 | Pour Point, °F (°C), 100.0 (38) |
| Distillation, °F (°C), D-1160 | Sulfur, XRF, wt.% 2.37 |
| IBP/5 693/760 (367/404) | Nitrogen, KJEL, wt.% 0.079 |
| 10/20 777/799 (414/426) | Hydrogen, wt.% 12.20 |
| 30/40 815/832 (435/444) | Chlorine, ppm <1.0 |
| 50/60 850/870 (454/465) | Carbon Residue, D-189, wt.% 0.14 |
| 70/80 894/920 (479/493) | Viscosity, SSU at 100°F (38°C) 319.0 |
| 90/95 958/979 (514/526) | Viscosity, SSU at 210°F (99°C) 51.1 |
| EP/%Rec. 1053/99.0 (567/99.0) | Specific Gravity 0.9200 |

TABLE III

| | Catalyst | Activity[1] | Selectivity[2] | Stability[3] |
|---|---|---|---|---|
| No. | Description of support | Reactor temp. to provide 60% conv. | Vol. % conv. to 300°—700° (149°—371°F) product | °F/day (°C/day) |
| 1 | 10% LZ-10 and 90% gamma alumina | 772°F (411°C) | 83.5 | 0.68 (0.38) |
| 2 | 10% LZ-10 and 90% $SiO_2$—$Al_2O_3$ in gamma alumina matrix | 750°F (399°C) | 83.5 | −0.72 (−0.40) |
| 3 | 5% LZ-10 and 95% $SiO_2$—$Al_2O_3$ in gamma alumina matrix | 776°F (413°C) | 87.4 | 0.09 (0.05) |
| 4 | 20% LZ-10 and 80% gamma alumina | 750°F (399°C) | 75.2 | 0.39 (0.22) |

[1]Activity data are those obtained on tenth day of run.

[2]Selectivity data are an average of data obtained over 10 days and are calculated as the volume of 300°—700°F (149°—371°C) components to the total volume of components boiling at or below 700°F (371°C).

[3]Stability data were calculated using the reactor temperature required to produce a 60% conversion on the 2nd and 10th days of the run.

The data in Table III reveal that in comparison to the two hydrogenation catalysts having supports consisting of LZ-10 and gamma alumina, the catalysts of the invention are far superior in terms of overall activity, selectivity, and stability. A comparison of Catalyst Nos. 1 and 2 shows that, for the same percentage of LZ-10 in the support, Catalyst No. 2 prepared in accordance with the invention was 22°F (12.2°C) more active and more stable than Catalyst No. 1. In addition, Catalyst No. 2 proved to be as selective for producing midbarrel products as Catalyst No. 1. Comparing Catalysts Nos. 2 and 4 and Catalysts 3 and 1 shows that the catalysts of the invention are as active, but substantially more stable and selective, than their LZ-10-alumina comparisons containing twice as much zeolite.

Example II

A second experiment was performed under the run conditions of Example I to demonstrate the improved performance attainable with the catalysts of the invention in comparison to the catalysts described in US—A—4,097,365. The catalysts utilized in the experiment were prepared as follows:

Catalyst No. 5

A catalyst support was prepared in the same manner as described in Example 3 of US—A—4,097,365 except that 54 parts of the silica-alumina graft copolymer were mixed with 46 parts of the hydrous alumina

gel. The support (in the size and shape as Catalyst No. 1) was calcined and impregnated with a nickel nitrate-ammonium metatungstate solution as in the preparation of Catalyst No. 1, and then dried and calcined in the same way. The final catalyst contained 4.1 wt.% nickel components (as NiO) and 24.4 wt.% tungsten components (as $WO_3$) supported on a base consisting essentially of a dispersion of 75/25 silica-alumina in an alumina matrix, the base having an overall silica content of 40% and an overall alumina content of 60%.

Catalyst No. 6

This catalyst was prepared in the same manner as Catalystf No. 5 except that LZ-10 in the ammonium form and peptized alumina binder were incorporated into the support such that, after calcination, LZ-10 in the hydrogen form comprised 10 percent by weight of the support and the binder comprised 20 percent by weight of the support.

The results obtained from testing Catalysts Nos. 5 and 6 for activity, selectivity, and stability are reported in Table IV.

TABLE IV

| Catalyst | | Activity[1] | Selectivity[2] | Stability[3] |
|---|---|---|---|---|
| No. | Description of support | Reactor temp. to provide 60% conv. | Vol. % conv. to 300°—700°F (149—371°C) product | °F/day (°C/day) |
| 5 | Dispersion of $SiO_2/Al_2O_3$ in gamma alumina matrix | 773°F (412°C) | 88.7 | 0.23 (0.13) |
| 6 | 10% LZ-10 and 90% dispersion of $SiO_2$—$Al_2O_3$ in gamma alumina | 753°F (401°C) | 87.4 | 0.05 (0.03) |

[1]Activity data are those obtained on tenth day of run.

[2]Selectivity data are an average of data obtained over 10 days and are calculated as the volume of 300°—700°F (149—371°C) components to the total volume of components boiling at or below 700°F (371°C).

[3]Stability data were calculated using the reactor temperatures required to produce a 60% conversion on the 2nd and 10th days of the run.

As is self-evident from the data in Table IV, the catalyst of the invention, Catalyst No. 6, proved far superior to a catalyst similar to that described in Example 3 of US—A—4,097,365. Catalyst No. 6 provides substantially more activity and stability than Catalyst No. 5 with no significant loss in selectivity.

Example III

A third comparison experiment was run to determine the activity of two catalysts of the invention comprising nickel and molybdenum components on supports comprising LZ-10 and a dispersion of silica-alumina in a gamma alumina matrix versus a catalyst comprising nickel and molybdenum components on a support comprising LZ-10 and alumina but containing no dispersion. The catalysts were prepared as follows:

Catalyst No. 7

Sixty grams of gamma alumina powder were comulled with 120 gm LZ-10 in the ammonia form, 20 gm peptized alumina, 75 gm ammonium heptamolybdate ($(NH_4)_6Mo_7O_{24} \cdot 4H_2O$), and 85 gm nickel nitrate hexahydrate. The mulled mixture was extruded through a die similar to that used in preparing Catalyst No. 1, cut into $\frac{1}{4}$—$\frac{1}{2}$ inch (0.64 to 1.27 cm) lengths, and calcined in air at 900°F (482°C). The resulting catalyst contained 7.7 wt.% nickel components (as NiO), 21.9 wt.% molybdenum components (as $MoO_3$), about 43 wt.% LZ-10, about 21 wt.% gamma alumina, and the remainder (about 7 wt.%) peptized alumina.

Catalyst No. 8

This catalyst was prepard in the same fashion as was Catalyst No. 7 except that, in place of the gamma alumina, 60 gm of a powdered dispersion of 75/25 silica-alumina in a gamma alumina matrix was used. The dispersion was prepared by spray drying a mixture comprising 33 parts by weight silica-alumina graft copolymer with 67 parts by weight of hydrous alumina gel. The final catalyst contained 7.4 wt.% nickel components (as NiO), 21.5 wt.% molybdenum components (as $MoO_3$), about 43 wt.% LZ-10, about 7% of peptized alumina, and about 21 % of the dispersion containing 25 wt.% silica and 75 wt.% alumina overall.

Catalyst No. 9

This catalyst was prepared in the same manner as Catalyst No. 8 except that the dispersion was prepared by mixing 54 parts by weight of 75/25 silica-alumina graft copolymer with 46 parts by weight of hydrous alumina gel. The final catalyst was of the same composition as Catalyst No. 8 except for the overall silica and alumina contents of the dispersion, which were 40% and 60% by weight, respectively.

The foregoing catalysts were subjected to the 10-day activity tests described in Example I, and the results are shown in Table V. As shown, the results prove the superiority of the catalysts of the invention (i.e., Catalysts Nos. 8 and 9) in all categories. In addition, the data show the improvement obtained when the silica contents of the catalysts of the invention are increased.

TABLE V

| Catalyst | | Activity[1] | Selectivity[2] | Stability[3] |
|---|---|---|---|---|
| No. | Description of support | Reactor temp. to provide 60% conv. | Vol. % conv. to 300°—700°F (149°—371°C) product | °F/day (°C/day) |
| 7 | LZ-10 and gamma alumina | 745°F (396°C) | 74.8 | 1.43 (0.79) |
| 8 | LZ-10 and $SiO_2$—$Al_2O_3$ in gamma alumina matrix (25% $SiO_2$ overall) | 743°F (395°C) | 79.4 | 0.17 (0.09) |
| 9 | LZ-10 and $SiO_2$—$Al_2O_3$ in gamma alumina matrix (40% $SiO_2$ overall) | 733°F (389°C) | 79.5 | 0.88 (0.49) |

[1]Activity data are those obtained on tenth day of run.
[2]Selectivity data are an average of data obtained over 10 days and are calculated as the volume of 300°—700°F (149°—371°C) components to the total volume of components boiling at or below 700°F (371°C).
[3]Stability data were calculated using the reactor temperatures required to produce a 60% conversion on the 2nd and 10th days of the run.

Example IV

A fourth experiment was conducted to compare the catalytic properties of a catalyst of the invention incorporating a stabilized Y zeolite with the catalytic properties of a similar catalyst containing stabilized Y zeolite but containing no dispersion of silica-alumina in an alumina matrix. The two catalysts were prepared as follows:

Catalyst No. 10

A mixture of 40 gm stabilized Y zeolite (prepared in accordance with the method described in U.S. Patent No. 3,929,672 for Catalyst A in Example 16 but without adding palladium), 40 gm peptized alumina binder, and 120 gm of dispersion of the kind described for Catalyst No. 9 were comulled with a 380 ml aqueous solution containing 78 gm ammonium heptamolybdate tetrahydrate, 29.1 gm phosphoric acid (85% $H_3PO_4$), and 85 gm nickel hexahydrate. The resulting material was extruded in the same manner as Catalyst No. 1, cut into particles of $\frac{1}{4}$—$\frac{1}{2}$ inch (0.64 to 1.27 cm) length, and calcined in air at 900°F (482°C). The final catalyst contained about 6 wt.% nickel components (as NiO), about 19 wt.% molybdenum components (as $MoO_3$), and about 6 wt.% phosphorus components (as $P_2O_5$).

Catalyst No. 11

This catalyst was prepared in a manner similar to that of Catalyst No. 10 with the major difference being (1) that the comulled mixture was extruded through a die having circular openings therein of about $\frac{1}{16}$ inch (0.16 cm) diameter and (2) the comulled mixture contained 120 gm of powdered gamma alumina instead of the dispersion. The resulting catalyst had the same percentage composition of nickel, molybdenum, and phosphorus components as Catalyst No. 10.

The foregoing catalysts were then tested in a manner similar to that described in Example I except that Catalyst No. 10 was run for 13.6 days and Catalyst No. 11 for 8 days. The data obtained are presented in Table VI.

TABLE VI

| Catalyst | | Activity[1] | Selectivity[2] | Stability[3] |
|---|---|---|---|---|
| No. | Description of support | Reactor temp. to provide 60% conv. | Vol. % conv. to 300°—700°F (149°—371°C) product | °F/day (°C/day) |
| 10 | Stabilized Y plus $SiO_2$—$Al_2O_3$ in gamma alumina matrix | 733°F (390°C) | 70.0 | 1.1 for days 3.2 to 10 (0.61 for days 3.2 to 10) 0 for days 10.9 to 13.6 |
| 11 | Stabilized Y plus gamma alumina | 739°F (393°C) | 73.0 | 0.58 for days 2.8 to 8.1 (0.32 for days 2.8 to 8.1) |

[1]Activity as reported for Catalyst No. 10 is a corrected value obtained from data determined for the 10th day of the run, and the activity data reported for Catalyst No. 11 are extrapolated from data derived on the eighth day of the run.

[2]Selectivity data are the average of data obtained during the first 10 days with Catalyst No. 10 and the 8 days of run with Catalyst No. 11.

[3]Stability data were calculated using the reactor temperatures required to produce a 60% conversion on the days specified in the Table.

The results in Table VI again show the overall superiority of the catalyst of the invention (Catalyst No. 10) with respect to activity, selectivity, and stability. The 6°F (3.3°C) differential in activity between the catalyst of the invention and the comparison catalyst represents about a 20% improvement in activity. Especially significant is the fact that after 10.9 days, Catalyst No. 10 showed no signs of deactivation, and thus the high activity indicated by the 733°F (389°C) result could be expected to be maintained.

Example V

Catalyst No. 12 was prepared in the same manner as Catalyst No. 9 except that LZ-20 rather than LZ-10 was utilized. (LZ-20 is a crystalline aluminosilicate zeolite, available from Union Carbide, Linde Division, having a unit cell size, a water sorption capacity, a surface area, and an ion exchange capacity somewhat higher than that of LZ-10). The catalyst was tested in the same manner as described in Example I except that the run length was 8.5 days rather than 10 days. The results of the experiment were as follows: Activity: 733°F (390°C) (the operating temperature on the last day of run), Selectivity: 67.0% (as the average percentage conversion to middle distillates during the run), and Stability: 1.94°F/day (1.08°C/day) as calculated between 2.1 days and the end of run and 1.86°F/day (1.03°C/day) between 5.3 days and end of the run. A comparison of these data with those of Catalyst No. 9 in Example III indicate that better results are obtained with LZ-10 in the catalyst support of the invention than with LZ-20.

Example VI

Catalyst No. 13 was prepared by mulling a mixture consisting essentially of 140 gm of a dispersion of silica-alumina in an alumina matrix as was used to prepare Catalyst No. 5, 20 gm LZ-10 in ammonia form (i.e., as in Example I), and 40 gm peptized alumina with 380 ml of an aqueous solution containing 78 gm ammonium heptamolybdate tetrahydrate, 29.1 gm phosphoric acid (85% $H_3PO_4$), and 85 gm nickel nitrate hexahydrate. The mulled mixture was wet sufficiently to form a paste and extruded through a die similar to that described in Example I. The extrudate was cut into $\frac{1}{4}$—$\frac{1}{2}$ inch (0.64 to 1.27 cm) particles in length, dried, and calcined at 900°F (482°C). The finished catalyst contained 7.6 wt.% nickel components (as NiO), 21.2 wt.% molybdenum components (as $MoO_3$), and 7.1 wt.% phosphorus components (as $P_2O_5$).

The foregoing catalyst was tested for its catalytic properties in the same manner as described in Example I, and the results were as follows: Activity: 750°F (399°C) on the tenth day of run, Selectivity: 85.6 as the average over the ten days of operation, and Stability: 0.85°F/day (0.47°C/day) from days 2 through 10 and 0.11°F/day (0.06°C/day) from days 5.8 through 10. These results indicate the high activity, selectivity, and stability of this embodiment of the catalyst of the invention.

Example VII

During the runs performed on Catalyst No. 6 in Example II and Catalyst No. 10 in Example IV, samples of the product oils were obtained and analyzed for sulfur content by X-ray fluorescence analysis and nitrogen content by coulometric analysis. As shown by the data in Table II, the sulfur and nitrogen contents of the feedstock were 2.37 wt.% and 0.079 wt.%, respectively. In Table VII are reported the results of the analyses performed on the samples of product oil obtained in the experiments described in Examples II and IV. The data in Table VII indicate that Catalysts Nos. 6 and 10 had substantial activity for hydrodenitrogenation and hydrodesulfurization.

TABLE VII

| Catalyst | Temp. °F (°C) | Days into run when sample taken | Sulfur in product, ppmw | Nitrogen in product, ppmw |
|---|---|---|---|---|
| No. 6 | 750 (399) | 10 | 6 | — |
| No. 10 | 730 (388) | 10 | 93 | 2.2 |
| No. 10 | 734 (390) | 13 | 62 | 1.3 |

Example VIII

Zeolite LZ-10 is believed to be prepared by high temperature, steam calcining the hydrothermally stable and ammonia-stable Zeolite Y compositions described in US—A—3,929,672, herein incorporated by reference. One specific method by which LZ-10 may be prepared is as follows:

A sample of air dried ammonium exchanged Zeolite Y having the composition exclusive of water of hydration:

$$0.156\ Na_2O{:}0.849\ (NH_4)_2O{:}Al_2O_3{:}5.13\ SiO_2$$

is tableted into ½ inch (1.27 cm) diameter slugs and charged to a Vycor tube provided with external heating means and having a 24 inch (61 cm) length and a 2.5 inch (6.35 cm) diameter. The temperature of the charge is first raised to 600°C in about 0.25 hours and then held at this temperature for one hour. During this 1.25 hour period, a pure steam atmosphere at 14.7 psia (1 atm) generated from demineralized water is passed upwardly through the charge at a rate of 0.1 to 0.5 lbs/hr (45.4 to 227 gm/hr). Ammonia gas generated during the heating deammoniation of the zeolite is passed from the system continuously. At the termination of the heating period, the steam flow is stopped and the temperature of the charge is lowered to ambient room temperature over a period of five minutes. The charge is removed from the Vycor tube, and the sodium cation content of the steam material is reduced to about 0.25 weight percent (as $Na_2O$) by ion exchange using an aqueous solution of 30 weight percent ammonium chloride at reflux.

The low sodium material thus prepared is recharged to the Vycor tube and again steamed, this time using pure steam at 14.7 psia (1 atm) and a temperature of 800°C for 4 hours. The product is then cooled to ambient temperature and has the following typical characteristics: Surface Area = 530 $m^2$/gm, Adsorptive Capacity of water at 4.6 mm partial pressure and 25°C = 4.6 weight percent, and an ion exchange capacity equal to 4% of that of a sodium Y zeolite having a comparable $SiO_2{:}Al_2O_3$ ratio. (The comparable sodium Y zeolite to which LZ-10 zeolite is compared in the specification and claims herein is a sodium Y zeolite having essentially the same silica to alumina ratio as LZ-10 and having a sodium to aluminum content such that the ratio of $Na_2O{:}Al_2O_3$ is equal to 1.0).

Example IX

In this Example, the most preferred embodiment of the invention will be illustrated.

A gas oil feedstock, such as that shown in Table II, is passed through a reactor vessel of suitable industrial size and therein contacted at temperatures in the range of 700° to 750°F (371° to 399°C) and at a pressure of about 2000 psig (135 atm) with a sulfided catalyst consisting essentially of about 5.0% by weight nickel components, calculated as NiO, and 25.0% by weight tungsten components, calculated as $WO_3$, on a support consisting essentially of 40% LZ-10 zeolite in the hydrogen form and the balance a dispersion of a 75/25 silica-alumina dispersed in alumina, with the dispersion consisting of 44 parts silica-alumina and 56 parts alumina. Other conditions employed in the reactor vessel are: 5.0 LHSV space velocity and a hydrogen recycle rate of 5000 SCF/bbl (890.56 cc/ml) as measured at 60°F (15.6°C).

When operating in the foregoing manner, the overall activity, selectivity, and stability of the catalyst proves highly useful for the production of midbarrel products.

Industrial applicability

The contemplated use of the catalyst of the invention is for hydrocracking of gas oils and the like to produce midbarrel products boiling in the 300° to 700°F (149° to 371°C) range, and for such hydrocracking, the most preferred catalyst comprises sulfided nickel and tungsten components on a support consisting essentially of LZ-10 zeolite in the hydrogen form and a dispersion of silica-alumina in gamma alumina.

While particular embodiments of the invention have been described, it will be understood, of course, that the invention is not limited thereto since many obvious modifications can be made, and it is intended to include within this definition any such modifications as will fall within the scope of the appended claims.

Having now described the invention, I claim:

## Claims

1. A hydrocracking catalyst composition containing a support comprising in intimate heterogeneous mixture (1) a crystalline aluminosilicate zeolite having activity for catalytically cracking hydrocarbons and (2) silica-alumina in an alumina matrix, characterized by said support carrying at least one hydrogenation component selected from the group consisting of Group VIII and Group VIB metals, their oxides, and sulfides, and said silica-alumina in an alumina matrix comprising a dispersion of silica-alumina in a gamma alumina matrix, wherein said support exclusive of said zeolite comprises at least 75 weight percent of said dispersion.

2. A catalyst composition as defined in claim 1 wherein said crystalline aluminosilicate zeolite contains one or more components selected from the group consisting of hydrogen ions, hydrogen ion precursors, and multivalent metal-containing cations.

3. A catalyst composition as defined in either of claims 1 and 2 wherein said hydrogenation component is selected from the group consisting of platinum, palladium, cobalt, nickel, tungsten, molybdenum, their oxides and their sulfides.

4. A catalyst composition as defined in any one of claims 1—3 wherein said crystalline aluminosilicate zeolite contains less than 0.5 weight percent alkali metal components, calculated as alkali metal oxides.

5. A catalyst composition as defined in any one of claims 1—4 wherein said crystalline aluminosilicate zeolite is a modified Y zeolite having a $SiO_2$:$Al_2O_3$ ratio between 3.5 and 6, a surface area between 500 and 700 $m^2$/gm, and an ion exchange capacity, when in the sodium form, less than 20 percent that of a sodium Y zeolite of comparable $SiO_2$:$Al_2O_3$ ratio.

6. A catalyst composition as defined in any one of claims 1—5 wherein said dispersion of silica-alumina particles in a gamma alumina matrix comprises between 20 percent and 65 percent by weight silica-alumina, and said silica-alumina contains between 50 percent and 90 percent by weight silica.

7. A catalyst composition as defined in any one of claims 1—6 wherein said crystalline aluminosilicate zeolite is ion exchanged to contain one or more rare earth metal ions.

8. A catalyst composition as defined in any one of claims 1—7 wherein said crystalline aluminosilicate zeolite has a water absorption capacity at 4.6 mm water vapor partial pressure and 25°C. of less than 8 percent by weight of the zeolite.

9. A catalyst composition as defined in any one of claims 1—8 further characterized in that said crystalline alumino-silicate zeolite has a unit cell size between 24.25 and 24.35 Angstroms.

10. A catalyst composition as defined in any one of claims 1—9 wherein said crystalline aluminosilicate zeolite is LZ-10 zeolite.

11. A catalyst composition as defined in any one of claims 1—10 wherein said crystalline aluminosilicate zeolite and said silica-alumina in an alumina matrix are held together in said support by a binder.

12. A catalyst composition as defined in any one of claims 1—11 wherein said support carries a Group VIB hydrogenation component selected from the group consisting of a molybdenum component and a tungsten component and a Group VIII hydrogenation component selected from the group consisting of a nickel component and a cobalt component.

13. A catalyst composition as defined in any one of claims 1—12 wherein said dispersion of silica-alumina particles in a gamma alumina matrix is prepard by mulling a dry silica-alumina cogel or a silica-alumina graft copolymer with an alumina hydrogel and subsequently spray drying the resultant mixture.

14. A catalyst composition as defined in any one of claims 1—13 wherein said crystalline aluminosilicate zeolite contains at least 50 percent of its pore volume in pores of diameter greater than 8 Angstroms.

15. A catalyst composition as defined by claim 12 wherein said Group VIB hydrogenation component is a tungsten component and said Group VIII hydrogenation component is a nickel component.

16. A catalyst composition as defined in any one of claims 1 to 15 wherein said support is made by the process comprising the steps of:

(1) calcining an ammonium-exchanged zeolite Y containing between 0.6 and 5 weight percent sodium, calculated as $Na_2O$, at a temperature between 600°F and 1650°F (316°C and 899°C) in contact with water vapor for a sufficient time to substantially reduce the unit cell size of said zeolite and bring it to a value between 24.40 and 24.64 Angstroms;

(2) subjecting the calcined zeolite to further ammonium ion exchange under conditions such that the sodium content of the zeolite is reduced below 0.6 weight percent, calculated as $Na_2O$;

(3) calcining the zeolite obtained from step (2) in contact with sufficient water vapor and for a sufficient time such that the unit cell size of the zeolite is reduced to between 24.25 and 24.35 Angstroms and the water absorption capacity of the zeolite at 4.6 mm water vapor partial pressure and 25°C is less than 8 percent by weight of the zeolite; and

(4) intimately admixing the zeolite obtained from step (3) with a dispersion of silica-alumina particles in a gamma alumina matrix, and calcining the resulting admixture.

17. A catalyst composition as defined in any one of claims 1—16 wherein said support consists of said crystalline aluminosilicate zeolite, said dispersion of silica-alumina particles in a gamma alumina matrix and a peptized alumina binder.

18. A catalyst composition as defined in any one of claims 1—17 wherein said catalyst comprises particles in the shape of a three-leaf clover.

19. A process for hydrocracking hydrocarbon feedstocks containing greater than 50 weight percent of their components boiling above 371 deg. C (700 deg. F) to produce middle distillate products boiling between about 149 deg. C (300 deg. F) and 371 deg. C (700 deg. F) by contacting said hydrocarbon feedstock at an elevated temperature and pressure in the presence of hydrogen with a catalyst composition containing a support comprising a dispersion of silica-alumina in a gamma alumina matrix and carrying at least one hydrogenation component selected from the group consisting of Group VIII and Group VIB metals their oxides and sulfides characterized in that said support contains an intimate heterogeneous mixture of said dispersion and a crystalline aluminosilicate zeolite having activity for catalytically cracking hydrocarbons.

20. A process according to claim 19, wherein the catalyst is a catalyst according to any one of claims 1 to 18.

**Patentansprüche**

1. Hydrokrack-Katalysatorzusammensetzung, die einen Träger enthält, der in einem innigen heterogenen Gemisch (1) einen kristallinen Aluminosilikat-Zeolithen enthält, der eine Aktivität für das katalytische Kracken von Kohlenwasserstoffen hat, und (2) Siliciumdioxid-Aluminiumoxid in einer Grundmasse aus Aluminiumoxid, dadurch gekennzeichnet, daß der Träger mindestens eine Hydrierkomponente trägt, die aus der Gruppe ausgewählt ist, die aus den Metallen der Gruppe VIII und der Gruppe VIB und deren Oxiden und Sulfiden besteht, und daß das Siliciumdioxid-Aluminiumoxid in einer Grundmasse aus Aluminiumoxid mindestens teilweise aus einer Dispersion von Siliciumdioxid-Aluminiumoxid in einer Grundmasse aus Gamma-Aluminiumoxid besteht, wobei der Träger mit Ausnahme des genannten Zeolithen zu mindestens 75 Gew.-% aus der genannten Dispersion besteht.

2. Katalysatorzusammensetzung nach Anspruch 1, dadurch gekennzeichnet, daß der kristalline Aluminosilikat-Zeolith eine oder mehrere Komponenten enthält, die aus der Gruppe ausgewählt sind, die aus Wasserstoffionen, Vorstufen von Wasserstoffionen und mehrwertige Metalle enthaltenden Kationen besteht.

3. Katalysatorzusammensetzung nach Anspruch 1 oder 2, dadurch gekennzeichnet, daß die Hydrierkomponente aus der Gruppe ausgewählt ist, die aus Platin, Palladium, Kobalt, Nickel, Wolfram, Molybdän und deren Oxiden und Sulfiden besteht.

4. Katalysatorzusammensetzung nach einem der Ansprüche 1—3, dadurch gekennzeichnet, daß der kristalline Aluminosilikat-Zeolith weniger als 0,5 Gew.-% Alkalimetallkomponenten, als Alkalimetalloxide berechnet, enthält.

5. Katalysatorzusammensetzung nach einem der Ansprüche 1 bis 4, dadurch gekennzeichnet, daß der Zeolith ein modifizierter Zeolith vom Typ Y ist, der ein $SiO_2Al_2O_3$-Verhältnis zwischen 3,5 und 6 und eine spezifische Oberfläche zwischen 500 und 700 $m^2/g$ und in der Natriumform eine Ionenaustauschkapazität besitzt, die weniger als 20% der eines Natrium-Zeolithen vom Typ Y mit einem vergleichbaren $SiO_2:Al_2O_3$-Verhältnis beträgt.

6. Katalysatorzusammensetzung nach einem der Ansprüche 1 bis 5, dadurch gekennzeichnet, daß die Dispersion von Siliciumdioxid-Aluminiumoxid in einer Grundmasse aus Gamma-Aluminiumoxid zwischen 20 und 65 Gew.-% Siliciumdioxid-Aluminiumoxid enthält und daß das Siliciumdioxid-Aluminiumoxid zwischen 50 und 90 Gew.-% Siliciumdioxid enthält.

7. Katalysatorzusammensetzung nach einem der Ansprüche 1 bis 6, dadurch gekennzeichnet, daß der kristalline Aluminosilikat-Zeolith infolge eines Ionenaustausches ein oder mehrere Ionen von Seltenerdmetallen enthält.

8. Katalysatorzusammensetzung nach einem der Ansprüche 1 bis 7, dadurch gekennzeichnet, daß der Zeolith unter einem Wasserdampfpartialdruck von 4,6 mm und 25°C eine Wasserabsorptionskapazität von weniger als 8 Gew.-% des Zeolithen hat.

9. Katalysatorzusammensetzung nach einem der Ansprüche 1 bis 8, dadurch gekennzeichnet, daß der kristalline Aluminosilikat-Zeolith eine Elementarzellengröße zwischen 2,425 bis 2,435 nm hat.

10. Katalysatorzusammensetzung nach einem der Ansprüche 1 bis 9, dadurch gekennzeichnet, daß der kristalline Aluminosilikat-Zeolith ein Zeolith vom Typ LZ-10 ist.

11. Katalysatorzusammensetzung nach einem der Ansprüche 1 bis 10, dadurch gekennzeichnet, daß der kristalline Aluminosilikat-Zeolith und das Siliciumdioxid-Aluminiumoxid in einer Grundmasse aus Aluminiumoxid in dem Träger durch ein Bindemittel zusammengehalten werden.

12. Katalysatorzusammensetzung nach einem der Ansprüche 1 bis 11, dadurch gekennzeichnet, daß der Träger eine Hydrierkomponente der Gruppe VIB trägt, die aus der Gruppe ausgewählt ist, die aus einer Molybdänkomponente und einer Wolframkomponente besteht, sowie eine Hydrierkomponente der Gruppe VIII, die aus der Gruppe ausgewählt ist, die aus einer Nickelkomponente und einer Kobaltkomponente besteht.

13. Katalysatorzusammensetzung nach einem der Ansprüche 1 bis 12, dadurch gekennzeichnet, daß die Dispersion von Teilchen aus Siliciumdioxid-Aluminiumoxid in einer Grundmasse aus Gamma-Aluminiumoxid erzeugt wird, indem ein trockenes Siliciumdioxid-Aluminiumoxid-Mischgel oder ein

Siliciumdioxid-Aluminiumoxid-Pfropfcopolymer mit einem Aluminiumoxidhydrogel in einem Kollergang gemahlen wird.

14. Katalysatorzusammensetzung nach einem der Ansprüche 1 bis 13, dadurch gekennzeichnet, daß mindestens 50% des Porenvolumens des kristallinen Aluminosilikat-Zeolithen aus Poren besteht, die größer als 0,8 nm sind.

15. Katalysatorzusammensetzung nach Anspruch 12, dadurch gekennzeichnet, daß die Hydrierkomponente der Gruppe VIB eine Wolframkomponente und die Hydrierkomponente der Gruppe VIII eine Nickelkomponente ist.

16. Katalysatorzusammensetzung nach einem der Ansprüche 1 bis 15, dadurch gekennzeichnet, daß der Träger in einem Verfahren erzeugt wird, des folgende Schritte umfaßt:

(1) ein einem Ammoniumaustausch unterworfener Zeolith vom Typ Y, der zwischen 0,6 und 5 Gew.-% Natrium, als $Na_2O$ berechnet, enthält, wird bei einer Temperatur zwischen 316 und 899°C in Berührung mit Wasserdampf so lange kalziniert, daß die Elementarzellengröße des Zeolithen auf einen Wert zwischen 1,440 und 2,464 nm vermindert wird,

(2) der kalzinierte Zeolith wird erneut einem Ammoniumionenaustausch unter solchen Bedingungen unterworfen, daß der Natriumgehalt des Zeolithen unter 0,6 Gew.-% als $Na_2O$ berechnet, vermindert wird,

(3) der im Schritt (2) erhaltene Zeolith wird in Berührung mit einer genügenden Menge Wasserdampf so lange kalziniert, daß die Elementarzellengöße des Zeolithen auf einen Wert zwischen 2,425 und 2,435 nm vermindert wird und daß der Zeolith bei 25°C unter einem Wasserdampfpartialdruck von 4,6 mm eine Wasserabsorptionskapazität unter 8 Gew.-% des Zeolithen hat, und

(4) der im Schritt (3) erhaltene Zeolith wird mit einer Dispersion von Siliciumdioxid-Aluminiumoxid in einer Grundmasse aus Gamma-Aluminiumoxid innig vermischt, worauf das erhaltene Gemisch kalziniert wird.

17. Katalysatorzusammensetzung nach einem der Ansprüche 1 bis 16, dadurch gekennzeichnet, daß der Träger aus dem kristallinen Aluminosilikat-Zeolithen, der Dispersion von Siliciumdioxid-Aluminiumoxid-Teilchen in einer Grundmasse aus Gamma-aluminiumoxid, und einem Bindemittel aus peptisiertem Aluminiumoxid besteht.

18. Katalysatorzusammensetzung nach einem der Ansprüche 1 bis 17, dadurch gekennzeichnet, daß der Katalysator wenigstens teilweise aus Teilchen in Form von dreizähligen Kleeblättern besteht.

19. Verfahren zum Hydrokracken von Kohlenwasserstoff-Binsatzgut, dessen Komponenten zu mehr als 50 Gew.-% über 371°C sieden, wobei Mitteldestillatprodukte erhalten werden, die zwischen etwa 149°C und 371°C sieden, und wobei des Kohlenwasserstoff-Einsatzgut bei erhöhter Temperatur und erhöhtem Druck in Gegenwart von Wasserstoff mit einer Katalysatorzusammensetzung in Berührung gebracht wird, die einen Träger enthält, der wenigstens teilweise aus einer Dispersion von Siliciumoxid-Aluminiumoxid in einer Grundmasse aus Gamma-Aluminiumoxid besteht und mindestens einen Hydrierkomponente trägt, die aus der Gruppe ausgewählt ist, die aus den Metallen der Gruppe VIII und der Gruppe VIB, deren Oxiden und Sulfiden besteht, dadurch gekennzeichnet, daß der Träger ein inniges heterogenes Gemisch der genannten Dispersion und eines kristallinen Aluminosilikat-Zeolithen mit einer Aktivität zum katalytischen Kracken von Kohlenwasserstoffen enthält.

20. Verfahren nach Anspruch 19, dadurch gekennzeichnet, daß der Katalysator ein Katalysator nach einem der Ansprüche 1 bis 18 ist.

**Revendications**

1. Composition de catalyseur d'hydrocraquage contenant un support, comprenant en mélange hétérogène intime (1) un alumino-silicate zéolitique cristallin doué d'activité pour le craquage catalytique des hydrocarbures et (2) de la silice-alumine dans une matrice d'alumine, caractérisée en ce que ledit support porte au moins un composant d'hydrogénation choisi parmi les métaux des groupes VIII et VIB, leurs oxydes et leurs sulfures et ladite silice-alumine dans une matrice d'alumine comprend une dispersion de silice-alumine dans une matrice d'alumine γ, ledit support à l'exception de ladite zéolite comprenant au moins 75% en poids de ladite dispersion.

2. Composition de catalyseur selon la revendication 1, dans laquelle ledit alumino-silicate cristallin zéolitique contient un ou plusieurs composants choisis parmi les ions hydrogène, les précurseurs d'ions d'hydrogène et les cations contenant des métaux polyvalents.

3. Composition catalytique selon l'une quelconque des revendications 1 et 2, dans laquelle ledit composant d'hydrogénation est choisi parmi le platine, le palladium, le cobalt, le nickel, le tungstène, le molybdène, leurs oxydes et leurs sulfures.

4. Composition de catalyseur selon l'une quelconque des revendications 1 à 3, dans laquelle ledit alumino-silicate cristallin zéolitique contient moins de 0,5% en poids de composants de métaux alcalins, calculés en oxydes de métaux alcalins.

5. Composition de catalyseur selon l'une quelconque des revendications 1 à 4, dans laquelle ledit alumino-silicate cristallin zéolitique est une zéolite Y modifiée ayant un rapport $SiO_2/Al_2O_3$ compris entre 3,5 et 6, une surface spécifique comprise entre 500 et 700 $M^2/g$ et une capacité d'échange d'ions, lorsqu'elle est sous la forme sodium, de moins de 20% de celle d'une zéolite Y de sodium ayant un rapport $SiO_2/Al_2O_3$ comparable.

6. Composition de catalyseur selon l'une quelconque des revendications 1 à 5, dans laquelle ladite dispersion de particules de silice-alumine dans une matrice d'alumine γ comprend entre 20 et 65% en poids de silice-alumine et ladite silice-alumine contient entre 50 et 90% en poids de silice.

7. Composition de catalyseur selon l'une quelconque des revendications 1 à 6, dans laquelle ledit alumino-silicate cristallin zéolitique est substitué par échange d'ions pour contenir un ou plusieurs ions de métaux des terres rares.

8. Composition de catalyseur selon l'une quelconque des revendications 1 à 7, dans laquelle ledit alumino-silicate cristallin zéolitique a une capacité d'absorption d'eau de moins de 8% en poids de la zéolite sous une pression partielle de vapeur d'eau de 4,6 mm et à 25°C.

9. Composition de catalyseur selon l'une quelconque des revendications 1 à 8, caractérisée en ce que ledit alumino-silicate cristallin zéolitique a une dimension de cellule unitaire comprise entre 24,25 et 24,35 Å.

10. Composition de catalyseur selon l'une quelconque des revendications 1 à 9, dans laquelle ledit alumino-silicate cristallin zéolitique est la zéolite LZ-10.

11. Composition de catalyseur selon l'une quelconque des revendications 1 à 10, dans laquelle ledit alumino-silicate cristallin zéolitique et ladite silice-alumine dans une matrice d'alumine sont maintenus ensemble dans ledit support par un liant.

12. Composition de catalyseur selon l'une quelconque des revendications 1 à 11, dans laquelle ledit support porte un composant d'hydrogénation du groupe VIB choisi dans le groupe formé par un composant de molybdène et un composant de tungstène, et un composant d'hydrogénation du groupe VIII choisi dans le groupe formé par un composant de nickel et un composant de cobalt.

13. Composition de catalyseur selon l'une quelconque des revendications 1 à 12, dans laquelle ladite dispersion de particules de silice-alumine dans une matrice d'alumine γ est préparée par malaxage d'un cogel de silice-alumine sec ou d'un copolymère greffé de silice-alumine avec un hydrogel d'alumine puis par séchage par pulvérisation du mélange résultant.

14. Composition de catalyseur selon l'une quelconque des revendications 1 à 13, dans laquelle ledit alumino-silicate cristallin zéolitique contient au moins 50% de son volume poreux dans des pores de diamètre supérieur à 8 Å.

15. Composition de catalyseur selon la revendication 12, dans laquelle ledit composant d'hydrogénation du groupe VIB est un composant de tungstène et ledit composant d'hydrogénation du groupe VIII est un composant de nickel.

16. Composition de catalyseur selon l'une quelconque des revendications 1 à 15, dans laquelle ledit support est obtenu par le procédé comprenant les étapes suivantes:

(1) on calcine une zéolite Y substituée par échange avec des ions ammonium, contenant entre 0,6 et 5% en poids de sodium, calculé en $Na_2O$, à une température de 316 à 899°C (600 à 1 650°F) avec de la vapeur d'eau durant une durée suffisante pour réduire sensiblement la dimension de cellule unitaire de ladite zéolite et l'amener à une valeur comprise entre 24,40 et 24,64 Å;

(2) on soumet la zéolite calcinée à un nouvel échange avec des ions ammonium dans des conditions telles que la teneur en sodium de la zéolite soit réduite au-dessous de 0,6% en poids, calculée en $Na_2O$;

(3) on calcine la zéolite obtenue à l'étape (2) en contact avec suffisamment de vapeur d'eau et pendant une durée suffisante pour que la dimension de cellule unitaire de la zéolite soit réduite entre 24,25 et 24,35 Å et que la capacité d'absorption d'eau de la zéolite soit de moins de 8% en poids de la zéolite sous une pression partielle de vapeur d'eau de 4,6 mm et à 25°C; et

(4) on mélange intimement la zéolite obtenue à l'étape (3) avec une dispersion de particules de silice-alumine dans une matrice d'alumine γ et on calcine le mélange résultant.

17. Composition de catalyseur selon l'une quelconque des revendications 1 à 16, dans laquelle ledit support consiste en ledit alumino-silicate cristallin zéolitique, en ladite dispersion de particules de silice-alumine dans une matrice d'alumine γ et en un liant d'alumine peptisé.

18. Composition de catalyseur selon l'une quelconque des revendications 1 à 17, dans laquelle ledit catalyseur comprend des particules sous forme d'un trêfle à trois feuilles.

19. Procédé pour l'hydrocraquage de charges hydrocarbonées dont plus de 50% en poids de leurs composants bouent au-dessus de 371°C (700°F), pour produire des produits constituant des distillats moyens bouillant entre environ 149°C (300°F) et 371°C (700°F) par mise en contact de ladite charge hydrocarbonee à temperature et pression élevées en présence d'hydrogène avec une composition de catalyseur contenant un support comprenant une dispersion de silice-alumine dans une matrice d'alumine γ et portant au moins un composant d'hydrogénation choisi dans le groupe formé par les métaux du groupe VIII et du groupe VIB, leurs oxydes et leurs sulfures, caractérisé en ce que ledit support contient un mélange hètérogène intime de ladite dispersion et d'un alumino-silicate cristallin zéolitique ayant une activité pour craquer catalytiquement les hydrocarbures.

20. Procédé selon la revendication 19, dans lequel le catalyseur est un catalyseur selon l'un quelconque des revendications 1 à 18.